# EUROPEAN PATENT APPLICATION

(11) **EP 4 428 171 A1**
(43) Date of publication of application: **11.09.2024**
(21) Application number: 22889869.8
(22) Date of filing: 27.10.2022
(51) Int. Cl.: C08G 18/80, C07C 271/20, C08G 71/04

(54) **PRODUCTION METHOD FOR FLUORINE-CONTAINING POLYURETHANE**

(30) Priority: 02.11.2021 JP 2021179746
(71) Applicant: National University Corporation Kobe University, Kobe-shi, Hyogo 657-8501 (JP); AGC INC., Chiyoda-ku, Tokyo 1008405 (JP)
(72) Inventor: TSUDA, Akihiko, Kobe-shi, Hyogo 657-8501 (JP); OKAZOE, Takashi, Tokyo 100-8405 (JP); WADA, Hiroshi, Tokyo 100-8405 (JP); TANAKA, Hideaki, Tokyo 100-8405 (JP); SUNAYAMA, Yoshitaka, Tokyo 100-8405 (JP); KAKIUCHI, Toshifumi, Tokyo 100-8405 (JP)
(74) Representative: TBK
(86) International application number: PCT/JP2022/040118
(87) International publication number: WO 2023/080049

(57) **Abstract**

The objective of the present invention is to provide a method for producing a high quality fluorine-containing polyurethane without using an isocyanate, and a fluorine-containing biscarbamate compound used for the method. The method for producing a fluorine-containing polyurethane according to the present invention is characterized in comprising the step of reacting a fluorine-containing biscarbamate compound represented by the following formula (I) and a diol compound represented by the following formula (II), wherein the fluorine-containing polyurethane is represented by the following formula (III): 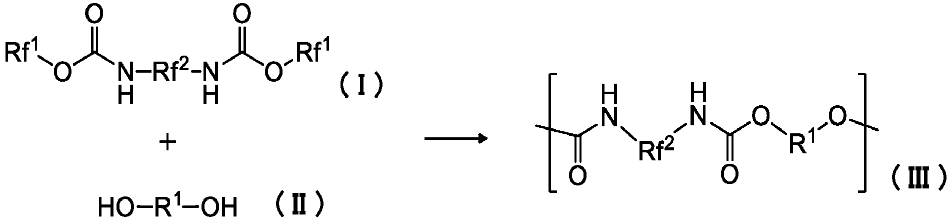 wherein Rf¹ is a fluorine-containing C₁₋₆ alkyl group, Rf² is a divalent fluorine-containing organic group, R¹ is a divalent organic group.

## Description

### TECHNICAL FIELD

The present invention relates to a method for producing a high quality fluorine-containing polyurethane without using an isocyanate, and a fluorine-containing biscarbamate compound used for the method.

### BACKGROUND ART

Polyurethane has been extensively used as a raw material for paint, an adiabatic material, a mat, a cushion or the like, and is generally industrially produced using an isocyanate as a raw material. For example, Non-patent document 1 discloses methods for producing polyurethane from a diisocyanate compound and a diol compound.

On the one hand, the use of an isocyanate is beginning to be regulated in Europe due to the high toxicity thereof. This movement is predicted to spread to Japan and the rest of the world in the future. In addition, an isocyanate is produced with using highly toxic phosgene.

An alternative synthesis method excellent environmentally and industrially for polyurethane has not been found regardless of such a situation. For example, it has been known that polyurethane can be synthesized from a biscarbamate compound and a diol compound in the presence or absence of a base. Such a biscarbamate compound is said to be a blocked isocyanate, and the isocyanate group is exposed to be used during a curing reaction.

A blocked isocyanate produced using an aliphatic alcohol as a blocking agent is transformed into a diisocyanate by heating. Since the heating temperature is a hundred and several tens of degrees centigrade or higher (Non-patent document 2), polyurethane may be possibly colored and a diisocyanate compound may be possibly decomposed. Also, Patent document 1 discloses a blocked isocyanate produced using a fluorine-containing aliphatic alcohol, and the blocked isocyanate is transformed into a diisocyanate compound under a temperature of a hundred and several tens of degrees centigrade or higher, such as 200°C.

A blocked isocyanate produced using an aromatic alcohol as a blocking agent may be transformed into a diisocyanate compound under relatively low temperature (Non-patent document 2), but the produced aromatic alcohol cannot be removed from polyurethane and thus the quality of the polyurethane may decrease.

Non-patent document 3 discloses a method for synthesizing polyurethane by transforming a blocked isocyanate into a diisocyanate compound under room temperature by using a fluoride ion as a catalyst. But this method is difficult to industrially use, since the reagent such as n-Bu₄NF is relatively expensive and cannot be easily removed from the produced polyurethane, and a fluoride ion itself is toxic.

### PRIOR ART DOCUMENT

### PATENT DOCUMENT

Patent document 1: WO 2011/125429

### NON-PATENT DOCUMENT

Non-patent document 1: R. Gosnell, et al., J. Macromol. Sci. -Phys., B1(4), pp. 831-850 (1967)
Non-patent document 2: NAGAKURA Minoru, Journal of the Japan Society of Color Material, 53(11), pp. 676-688 (1980)
Non-patent document 3: Madhu Sheri, et al., Angew. Chem. Int. Ed., 57, pp. 4599-4602 (2018)

### DISCLOSURE OF THE INVENTION

### PROBLEMS TO BE SOLVED BY THE INVENTION

An isocyanate has been used for a general industrial method for producing polyurethane as described above. In addition, a production method using a blocked isocyanate is considered as an alternative to this method. An isocyanate is used as a starting raw material compound for synthesizing a blocked isocyanate or a blocked isocyanate is intendedly transformed into an isocyanate to be reacted with a diol compound in such a production method. An isocyanate is, however, highly toxic and thus restrictions on the use thereof may be predicted to become increasingly severe in the future. In addition, the reaction involving the transformation of a blocked isocyanate into an isocyanate requires high temperature and thus the obtained polyurethane may become colored. Alternatively, an aromatic blocked isocyanate can be easily transformed into an isocyanate under relatively low temperature; but when an aromatic blocked isocyanate is transformed into an isocyanate, the produced aromatic alcohol may remain in polyurethane.

The objective of the present invention is to provide a method for producing a high quality fluorine-containing polyurethane without using an isocyanate, and a fluorine-containing biscarbamate compound used for the method under such a situation.

### MEANS FOR SOLVING THE PROBLEMS

The inventors of the present invention repeated intensive studies in order to solve the above-described problems. As a result, the inventors completed the present invention by finding that a fluorine-containing polyurethane can be produced under a relatively low temperature without detecting an isocyanate in the reaction mixture at least after the reaction and a fluorine-containing alcohol produced from the fluorine-containing biscarbamate compound hardly remain in the fluorine-containing polyurethane by reacting the specific fluorine-containing biscarbamate compound and the diol compound.

The present invention is hereinafter described.

[1] A method for producing a fluorine-containing polyurethane,
   the method comprising the step of reacting a fluorine-containing biscarbamate compound represented by the following formula (I) and a diol compound represented by the following formula (II),
   wherein the fluorine-containing polyurethane is represented by the following formula (III): 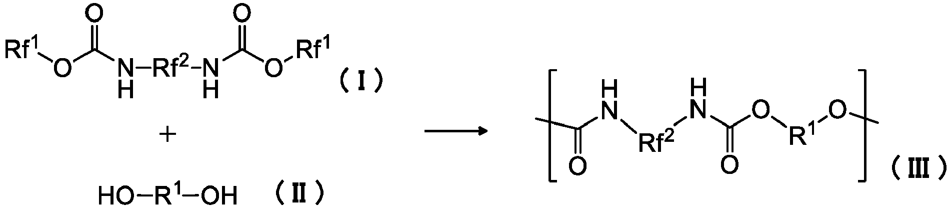 wherein
   Rf¹ is a fluorine-containing C₁₋₆ alkyl group,
   Rf² is a divalent fluorine-containing organic group,
   R¹ is a divalent organic group.
[2] The method according to the above [1], wherein the fluorine-containing biscarbamate compound represented by the formula (I) and the diol compound represented by the formula (II) are reacted under a temperature of 10°C or higher and 120°C or lower.
[3] The method according to the above [1] or [2], wherein the Rf² is a group represented by the formula: CH₂-Rf³-CH₂ wherein Rf³ is a fluorine-containing C₁₋₈ alkane-diyl group.
[4] The method according to the above [1] or [2], wherein the Rf² is a group represented by the formula: CH₂-(CF₂)ₙ-CH₂ wherein n is an integer of 1 or more and 8 or less.
[5] The method according to any one of the above [1] to [4], wherein the Rf¹ is CH(CF₃)₂.
[6] The method according to any one of the above [1] to [5], wherein the fluorine-containing biscarbamate compound represented by the formula (I) and the diol compound represented by the formula (II) are reacted in the presence of a base.
[7] A fluorine-containing biscarbamate compound represented by the following formula (I): 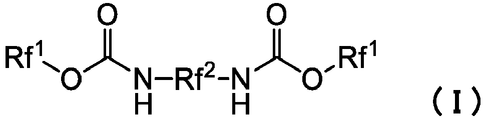 wherein
   Rf¹ is a fluorine-containing C₁₋₆ alkyl group,
   Rf² is a divalent fluorine-containing organic group.

### EFFECT OF THE INVENTION

Since the fluorine-containing biscarbamate compound used in the present invention can be reacted with a diol compound under a relatively low temperature, the produced fluorine-containing polyurethane is hardly colored. In addition, since the fluorine-containing alcohol produced from the fluorine-containing biscarbamate at the reaction has a relatively low boiling-point, the fluorine-containing alcohol can be easily removed from the fluorine-containing polyurethane as the target compound. Even if the fluorine-containing alcohol remains, preferred properties may be given to the fluorine-containing polyurethane. Furthermore, though the mechanism of the above-described reaction according to the present invention is not known, an isocyanate cannot be detected in the reaction mixture at least after the reaction. The present invention is, therefore, industrially very useful as the technology to safely produce a high quality fluorine-containing polyurethane

### BRIEF DESCRIPTION OF THE DRAWINGS

Figure 1 is an appearance photograph of the fluorine-containing polyurethane produced by the present invention method.
Figure 2 is an appearance photograph of the fluorine-containing polyurethane produced by the present invention method.
Figure 3 is an appearance photograph of the fluorine-containing polyurethane produced by the present invention method.
Figure 4 is an appearance photograph of the fluorine-containing polyurethane produced by the present invention method.

### MODE FOR CARRYING OUT THE INVENTION

The method for producing a fluorine-containing polyurethane according to the present invention comprises the step of reacting the mixture comprising the fluorine-containing biscarbamate compound represented by the formula (I) and the diol compound represented by the formula (II). The present invention method of the present invention is hereinafter specifically described, but the present invention is not restricted to the following specific examples. The "compound represented by the formula (x)" is abbreviated as the "compound (x)".

A method for producing polyurethane by using a biscarbamate compound called as a blocked isocyanate and transforming the compound into an isocyanate compound to be reacted with a diol compound by heating in the reaction mixture has been developed in the past as an alternative method for producing polyurethane by using an isocyanate compound. But high temperature is generally required for transforming a blocked isocyanate into an isocyanate compound, and such high temperature causes the coloration of polyurethane. A blocked isocyanate produced using an aromatic alcohol as a blocking agent has also been developed as a blocked isocyanate that can be transformed into an isocyanate compound at relatively low temperature. But since an aromatic alcohol is produced during the polymerization reaction and cannot be removed from polyurethane to be remained, the quality of the polyurethane is deteriorated.

On the one hand, the fluorine-containing biscarbamate compound (I) is used in the present invention. When the fluorine-containing biscarbamate compound (I) and the diol compound (II) are reacted, a chain fluorine-containing alcohol is produced as a side product but easy to remove in comparison with an aromatic alcohol. Even if a fluorine-containing alcohol remains in polyurethane, the fluorine-containing alcohol is clearly more difficult to oxidize than an aromatic alcohol, may not have a bad effect on the transparency of polyurethane, and may give preferred properties to polyurethane due to a fluoro group, such as repellency, an antifouling property, antiweatherability and abrasion resistance. In addition, high temperature is required for transforming a conventional blocked isocyanate into an isocyanate compound, but the fluorine-containing biscarbamate compound (I) according to the present invention can be reacted with the diol compound (II) even under a relatively low temperature.

The Rf¹ in the fluorine-containing biscarbamate compound (I) is independently a fluorine-containing C₁₋₆ alkyl group. The C₁₋₆ alkyl group means a monovalent linear or branched saturated aliphatic hydrocarbon group having a carbon number of 1 or more and 6 or less. An example of the C₁₋₆ alkyl group includes methyl, ethyl, n-propyl, isopropyl, n-butyl, 1-methylpropyl, 2-methylpropyl, 1,1-dimethylethyl, 2,2-dimethylethyl, n-pentyl, n-hexyl, 2-hexyl and 3-hexyl. The C₁₋₆ alkyl group is preferably a C₂₋₆ alkyl group and more preferably a C₂₋₄ alkyl group.

The substituent number of the fluoro group in the fluorine-containing C₁₋₆ alkyl group is not particularly restricted as long as the substitution is possible. The larger the number of the fluoro group is, the higher the reactivity of the fluorine-containing biscarbamate compound (I) becomes. Thus, the number is preferably 2 or more and more preferably 3 or more. With respect to the upper limit of the above-described substituent number, the number may be adjusted to, for example, 20 or less, and is preferably 15 or less. The C₁₋₆ alkyl group is preferably a sec-alkyl group or a tert-alkyl group. The Rf¹ is preferably -CH₂Rf⁴, -CH(Rf⁴)₂ or -C(Rf⁴)₃ wherein Rf⁴ is a C₁₋₅ perfluoro alkyl group and a plurality of Rf⁴ may be different from or the same as each other, and more preferably di(trifluoromethyl)methyl group [CH(CF₃)₂] or tri(trifluoromethyl)methyl group [C(CF₃)₃], of which all of the hydrogen atoms are substituted with fluoro groups at the carbon other than the 1^{st} position carbon. In addition, the fluorine-containing biscarbamate compound (I) may be substituted with a halogeno group selected from chloro, bromo and iodo as a similar electron withdrawing group in addition to fluoro.

The two Rf¹ in the fluorine-containing biscarbamate compound (I) may be the same as or different from each other and is preferably the same as each other.

The Rf² in the fluorine-containing biscarbamate compound (I) is a divalent fluorine-containing organic group. An example of the divalent fluorine-containing organic group includes a C₂₋₁₀ fluorine-containing alkane-diyl group and a divalent C₆₋₁₂ fluorine-containing aromatic group.

The C₂₋₁₀ alkane-diyl group in the C₂₋₁₀ fluorine-containing alkane-diyl group means a divalent linear or branched saturated aliphatic hydrocarbon group having a carbon number of 2 or more and 10 or less. An example of the C₂₋₁₀ alkane-diyl group includes ethane-diyl, n-propane-diyl, methylethanediyl, n-butane-diyl, methylpropane-diyl, n-pentane-diyl, n-hexane-diyl, n-heptane-diyl and n-octane-diyl. The C₂₋₁₀ alkane-diyl group is preferably a C₂₋₈ alkane-diyl group and more preferably a C₃₋₇ alkane-diyl group.

The substituent number of the fluoro group in the C₂₋₁₀ fluorine-containing alkane-diyl group is not particularly restricted as long as the substitution is possible. The larger the number of the fluoro group is, the higher the reactivity of the fluorine-containing biscarbamate compound (I) becomes. Thus, the number is preferably 2 or more and more preferably 3 or more. With respect to the upper limit of the above-described substituent number, the number may be adjusted to, for example, 20 or less, and is preferably 15 or less.

The α position carbon adjacent to the carbonate group (-NH-C(=O)-O-) in the fluorine-containing biscarbamate compound (I) is not preferably substituted with a fluoro group, and the group at the α position is preferably a methylene group (-CH₂-) or a methine group (>CH-). More specifically, the Rf² in the fluorine-containing biscarbamate compound (I) is preferably CH₂-Rf³-CH₂ wherein Rf³ is a C₁₋₈ fluorine-containing alkane-diyl group. The fluorine-containing biscarbamate compound (I) having the group as Rf² is more stable. The Rf² is more preferably the formula: CH₂-(CF₂)ₙ-CH₂ wherein n is an integer of 1 or more and 8 or less. The fluorine-containing biscarbamate compound (I) having the group as Rf² has more stable Rf² part and is more excellent in the reactivity.

The C₂₋₁₀ fluorine-containing alkane-diyl group may have an ether group (-O-). In other words, an example of the Rf² includes the group represented by the formula: -CH₂-Rf³-[-O-Rf⁶-]ₚ-O-Rf⁷-CH₂- wherein Rf⁵ to Rf⁷ are independently a C₁₋₄ fluorine-containing alkane-diyl group and the p is an integer of 0 or more and 100 or less. The Rf⁵ to Rf⁷ are preferably C₁₋₄ perfluoro alkane-diyl groups, and the p is preferably 50 or less. An example of the Rf⁵ to Rf⁷ independently includes CF₂, CF₂CF₂ and CF(CF₃).

An example of the divalent C₆₋₁₂ aromatic group in the divalent C₆₋₁₂ fluorine-containing aromatic group includes a divalent C₆₋₁₂ arylene group such as phenylene, naphthylene, indenylene and biphenylene; -Ph-O-Ph-, -Ph-CH₂-Ph-, -Ph-S-Ph-, -Ph-S(=O)-Ph- and -Ph-S(=O)₂-Ph-; and a divalent group formed by removing the hydrogen atoms in the terminal hydroxy groups of bisphenols such as Bisphenol A, Bisphenol AP, Bisphenol AF, Bisphenol B, Bisphenol BP, Bisphenol C, Bisphenol E, Bisphenol F, Bisphenol G, Bisphenol M, Bisphenol S, Bisphenol P, Bisphenol PH, Bisphenol TMC and Bisphenol Z.

All of the hydrogen atoms on the aromatic ring in the divalent C₆₋₁₂ fluorine-containing aromatic group may be substituted with fluoro, and the alkane-diyl group to bind 2 phenylene groups in bisphenols or the like may be also substituted with fluoro if possible.

For example, the fluorine-containing biscarbamate compound (I) can be produced by the following method. An isocyanate is not needed to be used in the following method.

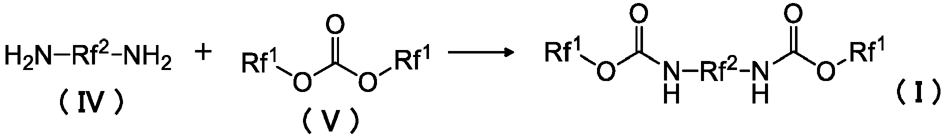

If the diamino compound (IV) and the carbonate compound (V) are commercially available, the commercial products may be used. Alternatively, the compounds may be synthesized. In particular, for example, the carbonate compound (V) can be synthesized by an ordinary method using phosgene and can be also synthesized by the reaction between a fluoro aliphatic hydrocarbon ester of trichloroacetic acid and a fluorine-containing alcohol or the method described in WO 2018/211953 without using phosgene.

For example, H₂N-CH₂-Rf³-CH₂-NH₂ wherein Rf³ is a C₁₋₈ fluorine-containing alkane-diyl group and H₂N-CH₂-(CF2)ₙ-CH₂-NH₂ wherein n is an integer of 1 or more and 8 or less can be used as the diamino compound (IV).

An example of the carbonate compound (V) includes bis(2,2,2-trifluoroethyl)carbonate, bis(2,2,3,3-tetrafluoropropyl)carbonate, bis(2,2,3,3,3-pentafluoropropyl)carbonate, bis(1,1,1,3,3,3-hexafluoroisopropyl)carbonate, bis(1,1,1,2,2,4,5,5,5-nonafluoro-4-trifluoromethyl-3-pentyl) carbonate, bis[1,1,1,3,3,3-hexafluoro-2-(trifluoromethyl)propane-2-yl]carbonate, bis(2,2,3,3,3-pentafluoropropyl)carbonate, bis(2,2,3,3,4,4,5,5-octafluoropentyl)carbonate and bis(2,2,3,3,4,4,5,5-octafluorocyclopentyl)carbonate.

The amounts of the diamino compound (IV) and the carbonate compound (V) may be appropriately adjusted. For example, a molar ratio of the carbonate compound (V) to the diamino compound (IV) is preferably adjusted to 2 times or more by mole, since 2 times by mole of the carbonate compound (V) theoretically reacts with the diamino compound (IV). The molar ratio may be adjusted to 5 times or more by mole and 20 times or less by mole. The molar ratio is preferably 15 times or less by mole and more preferably 10 times or less by mole.

A solvent may be used in the above-described reaction. The solvent is not particularly restricted. An example of the solvent includes a fluorine-containing solvent such as Asahiklin series manufactured by AGC, Novec series manufactured by 3M, Elnova series manufactured by TOKUYAMA METEL and 1,3-bis(trifluoromethyl)benzene; a nitrile solvent such as acetonitrile and benzonitrile; an ether solvent such as diethyl ether, glyme, diglyme, triglyme, tetraglyme, tetrahydrofuran and dioxane; a ketone solvent such as acetone and methyl ethyl ketone; an ester solvent such as ethyl acetate; a halogenated hydrocarbon solvent such as dichloromethane, chloroform and carbon tetrachloride; and an aromatic hydrocarbon solvent such as benzene, toluene and chlorobenzene. The solvent is preferably a fluorine-containing solvent excellent in the solubility of a fluorine-containing compound and a mixed solvent of a fluorine-containing solvent and the other solvent. When at least one of the diamino compound (IV) or the carbonate compound (V) is liquid in the reaction condition, a solvent may not be used. It is preferred not to use a solvent in terms of a cost and an environmental burden.

A base may be used in the above-described reaction. An example of the base includes an organic base such as pyridine, triethylamine, ethyldiisopropylamine, diazabicycloundecene (DBU), 1,4-diazabicyclo[2.2.2]octane (DABCO) and N-methylmorpholine; and an inorganic base such as sodium hydrogencarbonate, potassium hydrogencarbonate, sodium carbonate, potassium carbonate, cesium carbonate, calcium carbonate, sodium fluoride, potassium fluoride and cesium fluoride. An organic base is preferred in terms of the solubility in the reaction mixture and an appropriate basicity. In addition, an organic base may be recovered from the reaction mixture by distillation after the reaction in some cases. The usage amount of the base may be appropriately adjusted, and for example, 0.1 times or more by mole and 5 times or less by mole of the base to the diamino compound (IV) may be used. The base may not be used in terms of a cost and a residue.

The reaction condition may be appropriately adjusted. For example, the reaction temperature may be adjusted to 10°C or higher and 60°C or lower, and the reaction may be conducted under an atmospheric temperature. The reaction time may be also appropriately adjusted. For example, the reaction may be conducted until the consumption of at least one of the diamino compound (IV) and the carbonate compound (V) is confirmed by chromatography, NMR spectrum or the like, and the reaction time may be determined by a preliminary experiment. For example, the reaction time may be adjusted to 1 hour or more and 50 hours or less.

A general aftertreatment may be conducted after the reaction. For example, water and/or a non-water-miscible organic solvent such as a fluorine-containing solvent is added to the reaction mixture after the reaction, and the organic phase and the aqueous phase are separated. The thus obtained organic phase is dried over anhydrous sodium sulfate or anhydrous magnesium sulfate, and the solvent may be distilled away. The target compound may be further purified by chromatography or the like.

The R¹ in the diol compound (II) is a divalent organic group. An example of the R¹ includes a divalent C₂₋₁₀ chain aliphatic hydrocarbon group, a divalent C₃₋₁₀ cyclic aliphatic hydrocarbon group, a divalent C₆₋₁₅ aromatic hydrocarbon group, and a divalent organic group formed by binding 2 or more and 5 or less of the groups.

The "divalent C₂₋₁₀ chain aliphatic hydrocarbon group" means a divalent linear or branched saturated or unsaturated aliphatic hydrocarbon group having a carbon number of 2 or more and 10 or less. An example of the divalent C₂₋₁₀ chain aliphatic hydrocarbon group includes a C₂₋₁₀ alkane-diyl group, a C₂₋₁₀ alkene-diyl group and a C₂₋₁₀ alkyne-diyl group.

An example of the C₂₋₁₀ alkane-diyl group includes ethylene, n-propylene, isopropylene, n-butylene, 1-methylpropylene, 2-methylpropylene, 1,1-dimethylethylene, 2,2-dimethylethylene, n-pentylene, n-hexylene, n-heptylene, n-octylene and n-decylene. The C₂₋₁₀ alkane-diyl group is preferably a C₂₋₈ alkane-diyl group or a C₂₋₆ alkane-diyl group and more preferably a C₂₋₄ alkane-diyl group.

An example of the C₂₋₁₀ alkene-diyl group includes ethenylene (vinylene), 1-propenylene, 2-propenylene (allylene), butenylene, hexenylene, octenylene and decenylene. The C₂₋₁₀ alkene-diyl group is preferably a C₂₋₈ alkene-diyl group and more preferably a C₂₋₆ alkene-diyl group or a C₂₋₄ alkene-diyl group.

An example of the C₂₋₁₀ alkyne-diyl group includes ethynylene, propynylene, butynylene, hexynylene, octynylene and pentadecynylene. The C₂₋₁₀ alkyne-diyl group is preferably a C₂₋₈ alkyne-diyl group and more preferably a C₂₋₆ alkyne-diyl group or a C₂₋₄ alkyne-diyl group.

The "divalent C₃₋₁₀ cyclic aliphatic hydrocarbon group" means a divalent cyclic saturated or unsaturated aliphatic hydrocarbon group having a carbon number of 3 or more and 10 or less. The number of the ring may be 1 or 2 or more. An example of the group includes a C₃₋₁₀ cycloalkane-diyl group, a C₄₋₁₀ cycloalkene-diyl group and a C₄₋₁₀ cycloalkyne-diyl group. An example of the C₃₋₁₀ cycloalkane-diyl group includes cyclobutene-diyl, cyclopropane-diyl, cyclohexane-diyl and adamantane-diyl.

The "divalent C₆₋₁₅ aromatic hydrocarbon group" means a divalent aromatic hydrocarbon group having a carbon number of 6 or more and 15 or less. An example of the group includes phenylene, indenylene, naphthylene, biphenylene, phenanthrenylene and anthracenylene. The group is preferably a divalent C₆₋₁₂ aromatic hydrocarbon group and more preferably phenylene.

An example of the divalent organic group formed by binding 2 or more and 5 or less of the groups selected from the divalent C₂₋₁₀ chain aliphatic hydrocarbon group, the divalent C₃₋₁₀ cyclic aliphatic hydrocarbon group and the divalent C₆₋₁₅ aromatic hydrocarbon group includes a divalent C₃₋₁₀ cyclic aliphatic hydrocarbon group-divalent C₂₋₁₀ chain aliphatic hydrocarbon group, a divalent C₂₋₁₀ chain aliphatic hydrocarbon group-divalent C₃₋₁₀ cyclic aliphatic hydrocarbon group, a divalent C₆₋₁₅ aromatic hydrocarbon group-divalent C₂₋₁₀ chain aliphatic hydrocarbon group, a divalent C₂₋₁₀ chain aliphatic hydrocarbon group-divalent C₆₋₁₅ aromatic hydrocarbon group, a divalent C₂₋₁₀ chain aliphatic hydrocarbon group-divalent C₃₋₁₀ cyclic aliphatic hydrocarbon group-divalent C₂₋₁₀ chain aliphatic hydrocarbon group, a divalent C₃₋₁₀ cyclic aliphatic hydrocarbon group-divalent C₂₋₁₀ chain aliphatic hydrocarbon group-divalent C₃₋₁₀ cyclic aliphatic hydrocarbon group, and a divalent C₂₋₁₀ chain aliphatic hydrocarbon group-divalent C₆₋₁₃ aromatic hydrocarbon group-divalent C₂₋₁₀ chain aliphatic hydrocarbon group.

The above-described divalent organic group in the diol compound (II) may be substituted with 1 or more halogeno groups selected from fluoro, chloro, bromo and iodo. The halogeno group is preferably fluoro. The divalent C₃₋₁₀ cyclic aliphatic hydrocarbon group and the divalent C₃₋₁₀ cyclic aliphatic hydrocarbon group may have an ether group (-O-) and may be further substituted with a C₁₋₆ alkyl group in addition to the halogeno group. The substituent group is preferably fluoro. An example of the divalent C₃₋₁₀ cyclic aliphatic hydrocarbon group having an ether group includes a divalent group formed by removing the hydrogen atoms from the terminal hydroxy groups of isosorbide.

An example of the diol compound (II) includes ethanediol, propanediol, butanediol, pentanediol, hexanediol, heptanediol, octanediol and isosorbide. The groups may be substituted with the above-described halogeno group and may be substituted with fluoro.

An example of the R¹ in the diol compound (II) includes the divalent organic group represented by the following formula (VI) :

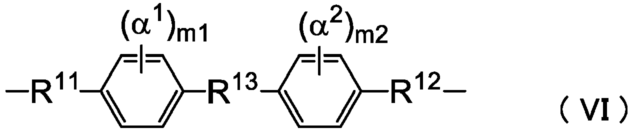

wherein
R¹¹ and R¹² are independently -(CR¹⁴R¹⁵)ₘ₃- or -(-O-(CR¹⁴R¹⁵)ₘ₄-)ₘ₅- wherein R¹⁴ and R¹⁵ are independently H or a C₁₋₆ alkyl group, m3 is an integer of 0 or more and 10 or less, m4 is an integer of 1 or more and 10 or less, m5 is an integer of 1 or more and 10 or less, and when m3 or m4 is an integer of 2 or more, a plurality of R¹⁴ and R¹⁵ are the same as or different from each other,
R¹³ is any one of the following divalent organic groups,

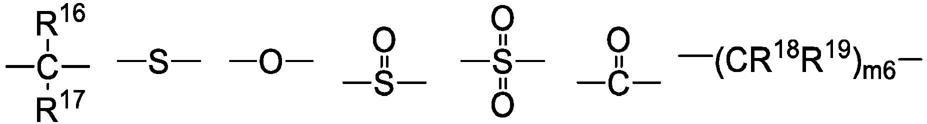

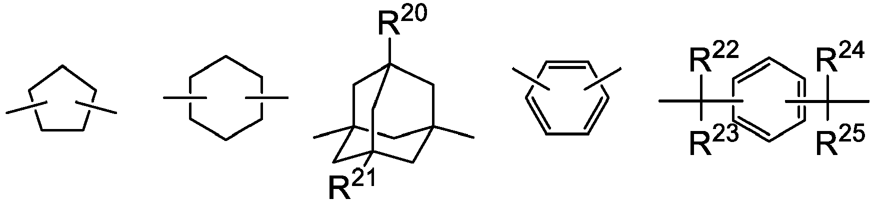

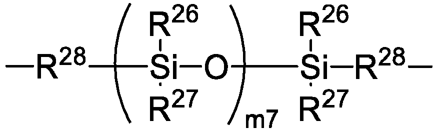

(wherein
R¹⁶ and R¹⁷ are independently H, a halogeno group, a C₁₋₂₀ aliphatic hydrocarbon group optionally having a substituent β, a C₁₋₂₀ alkoxy group optionally having a substituent β, a C₆₋₂₀ aromatic hydrocarbon group optionally having a substituent γ, or R¹⁶ and R¹⁷ are bound to form a C₃₋₂₀ carbon ring or a 5-12 membered hetero ring,
R¹⁸ and R¹⁹ are independently H or a C₁₋₆ alkyl group, and when m6 is an integer of 2 or more, a plurality of R¹⁸ and R¹⁹ may be the same as or different from each other,
R²⁰ to R²⁷ are independently a halogeno group, a C₁₋₂₀ aliphatic hydrocarbon group optionally having a substituent β, a C₁₋₂₀ alkoxy group optionally having a substituent β, or a C₆₋₁₂ aromatic hydrocarbon group optionally having a substituent Y,
R²⁸ is a C₁₋₉ alkane-diyl group optionally having a substituent β,
m6 is an integer of 1 or more and 20 or less,
m7 is an integer of 1 or more and 500 or less)
substituent α¹ and substituent α² are independently 1 or more substituent groups selected from the group consisting of a halogeno group, a C₁₋₂₀ aliphatic hydrocarbon group, a C₁₋₂₀ alkoxy group, a C₃₋₂₀ cycloalkyl group, a C₆₋₂₀ aromatic hydrocarbon group, a C₇₋₂₀ aralkyl group, a C₆₋₂₀ aromatic hydrocarbonoxy group and a C₃₋₂₀ cycloalkyl group,
m1 and m2 are independently an integer of 0 or more and 4 or less,
substituent β is 1 or more substituent groups selected from a C₁₋₆ alkoxy group, a C₁₋₇ acyl group, a halogeno group, a nitro group, a cyano group and a carbamoyl group,
substituent γ is 1 or more substituent groups selected from a C₁₋₆ alkyl group, a C₁₋₆ alkoxy group, a C₁₋₇ acyl group, a halogeno group, a nitro group, a cyano group and a carbamoyl group.

An example of the -Ph-R¹³-Ph- in the divalent organic group (VI) includes a divalent organic group formed by removing hydroxy groups from Bisphenol A, Bisphenol AP, Bisphenol AF, Bisphenol B, Bisphenol BP, Bisphenol C, Bisphenol E, Bisphenol F, Bisphenol G, Bisphenol S, Bisphenol TMC and Bisphenol Z.

An example of the - (CR¹⁴R¹⁵)ₘ₃- in the divalent organic group (VI) includes a single bond and a C₁₋₂ alkyl group, or an example of the - (-O-(CR¹⁴R¹⁵)ₘ₄-)ₘ₅- group includes -(-O-CH₂CH₂-)ₘ₅-, -(-O-CH(CH₃)CH₂-)ₘ₅- and -(-O-CH₂CH(CH₃)-)ₘ₅-.

In addition, an example of the R¹ in the diol compound (II) includes a divalent organic group represented by the formula - R²-[-X-R²-]ₘ- wherein X is O or S and preferably O, R² is a C₁₋₈ alkane-diyl group optionally substituted by a halogeno group, m in an integer of 1 or more and 180 or less, and when m is an integer of 2 or more, a plurality of X and R² may be respectively the same as or different from each other. The above-described halogeno group is preferably fluoro.

An example of the R² includes ethylene group (-CH₂CH₂-), propylene group [-CH(CH₃)CH₂- or -CH₂CH(CH₃)-] and tetramethylene group (-CH₂CH₂CH₂CH₂-). The groups may be substituted with a halogeno group, and the halogeno group is preferably fluoro.

The 'm' is preferably 5 or more, more preferably 10 or more, even more preferably 20 or more, and preferably 160 or less, more preferably 150 or less.

When the fluorine-containing biscarbamate compound (I) and the diol compound (II) are reacted, a solvent may be used. The solvent is not particularly restricted as long as the solvent is liquid under an atmospheric temperature and an ambient pressure and does not have a negative impact on the reaction. An example of the solvent includes a fluorine-containing solvent such as Asahiklin series manufactured by AGC, Novec series manufactured by 3M, Elnova series manufactured by TOKUYAMA METEL and 1,3-bis(trifluoromethyl)benzene; an aromatic hydrocarbon solvent such as benzene, toluene and chlorobenzene; a nitrile solvent such as acetonitrile and benzonitrile; an ether solvent such as diethyl ether, glyme, diglyme, triglyme, tetraglyme, tetrahydrofuran and dioxane; a ketone solvent such as acetone and methyl ethyl ketone; an ester solvent such as ethyl acetate; and a halogenated hydrocarbon solvent such as dichloromethane, chloroform and carbon tetrachloride. When at least one of the fluorine-containing biscarbamate compound (I) or the diol compound (II) is liquid in the reaction condition, a solvent may not be used. It is preferred not to use a solvent in terms of a cost and an environmental burden.

The usage amounts of the fluorine-containing biscarbamate compound (I) and the diol compound (II) may be appropriately adjusted. For example, the amount of the fluorine-containing biscarbamate compound (I) to be used may be adjusted to the same mole or the approximately same mole as the diol compound (II). For example, a molar ratio of one of the fluorine-containing biscarbamate compound (I) or the diol compound (II) to the other one may be adjusted to 0.8 or more and 1.2 or less. The molar ratio is preferably 0.9 or more, more preferably 0.95 or more, and preferably 1.1 or less, more preferably 1.05 or less. The above-described molar ratio may be adjusted to 1.

The fluorine-containing biscarbamate compound (I) and the diol compound (II) may be reacted in the presence of a base. An example of the base includes an organic base such as pyridine, triethylamine, ethyldiisopropylamine, diazabicycloundecene (DBU), 1,4-diazabicyclo[2.2.2]octane (DABCO) and N-methylmorpholine; and an inorganic base such as sodium hydrogencarbonate, potassium hydrogencarbonate, sodium carbonate, potassium carbonate, cesium carbonate, calcium carbonate, sodium fluoride, potassium fluoride and cesium fluoride. The base is preferably an organic base in terms of the solubility in the reaction mixture and an appropriate basicity. An organic base may be removed from the reaction mixture by distillation after the reaction in some cases. The usage amount of the base may be appropriately adjusted, and for example, the base may be used in an amount of 0.01 times or more by mole and 1 time or less by mole to the smaller number of mole of the fluorine-containing biscarbamate compound (I) or the diol compound (II). A base may not be used in terms of a cost and the residue thereof.

The reaction temperature may be appropriately adjusted and may be adjusted to, for example, 10°C or higher and 120°C or lower. The reaction may be conducted under an atmospheric temperature or with heating to reflux depending on the used solvent. The higher the reaction mixture is, the more successfully the reaction may proceed. The reaction temperature is, therefore, preferably 25°C or higher or 40°C or higher, more preferably 60°C or higher and even more preferably 80°C or higher. On the one hand, since the fluorine-containing polyurethane may be possibly colored due to excessively high reaction temperature in some cases, the reaction temperature is preferably 110°C or lower, more preferably 105°C or lower and even more preferably 100°C or lower.

The reaction time may be also appropriately adjusted. The reaction may be conducted until the consumption of at least one of the fluorine-containing biscarbamate compound (I) and the diol compound (II) is confirmed by chromatography, NMR spectrum or the like. The reaction time may be determined by a preliminary experiment. For example, the reaction time may be adjusted to 1 hour or more and 200 hours or less.

A general aftertreatment may be conducted after the reaction. For example, since the fluorine-containing polyurethane is often obtained as a solid or a viscous liquid, the solvent may be distilled away after the reaction. In addition, the fluorine-containing polyurethane may be washed using a solvent.

The inventors of the present invention experimentally found that an isocyanate is not detected at least in the reaction mixture to react the fluorine-containing biscarbamate compound (I) and the diol compound (II) after the reaction. Thus, the fluorine-containing polyurethane can be produced easily, safely and efficiently without using a highly toxic isocyanate compound by the present invention method. In addition, the fluorine-containing polyurethane produced by the present invention is excellent in heat resistance, flexibility, chemical resistance, repellency or the like due to the chain organic group having a fluoro group as a substituent group in the main skeleton. Furthermore, the quality of the polyurethane may further become high due to the remaining fluorine-containing alcohol.

### EXAMPLES

The present application claims the benefit of the priority date of Japanese patent application No. 2021-179746 filed on November 2, 2021. All of the contents of the Japanese patent application No. 2021-179746 filed on November 2, 2021, are incorporated by reference herein.

Hereinafter, the examples are described to demonstrate the present invention more specifically, but the present invention is in no way restricted by the examples, and the examples can be appropriately modified to be carried out within a range that adapts to the contents of this specification. Such a modified example is also included in the range of the present invention.

### Example 1: Synthesis of bis(1,1,1,3,3,3-hexafluoropropane-2-yl) (2,2,3,3,4,4-hexafluoropentane-1,5-diyl)dicarbamate [6FPBC]

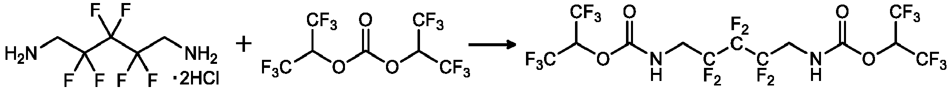

Bis(1,1,1,3,3,3-hexafluoropropane-2-yl)carbonate (BHFC) (33.9 mmol, 12.3 g), 2,2,3,3,4,4-hexafluoropentane-1,5-diamine hydrochloride (6FPDA·2HCl) (5.7 mmol, 1.6 g), triethylamine (12.9 mmol, 1.8 mL) and a hydrofluoroether solvent ("Novec^{™} 7100" manufactured by 3M, 34 mL) as a solvent were added to a 100 mL round-bottom flask, and the mixture was stirred at room temperature for 2.5 hours. Then, 1 M hydrochloric acid, chloroform, the above hydrofluoroether solvent and water were added to the reaction mixture, and the organic phase and the aqueous phase were separated. The thus obtained organic phase was dried over anhydrous sodium sulfate. The solvent was distilled away under reduced pressure, and the residue was dried in vacuo at 70°C for 2 hours to obtain a white solid as the target compound (1.37 g, 2.3 mmol, yield: 40%).

¹H NMR (400 MHz, CDCl₃, 293 K): δ/ppm: 5.68(sep, J=6.0Hz, 2H, CH), 5.41(t, J=6.6Hz, 2H, NH), 3.98(td, J=15.2, 6.4Hz, 4H, CH₂) ¹³C NMR (100 MHz, DMSO-d₆, 293 K): δ/ppm: 153.2, 120.9(q, ¹J_{C-F}=280.7Hz), 115.6(tt, ¹J_{C-F}=254.4Hz, ²J_{C-F}=32Hz), 111.0(m), 67.0(m), 41.1(m)

¹⁹F NMR (376 MHz, CDCl₃, 293 K, C₆F₆ as external standard) : δ/ppm: -73.6(d, J=6.8Hz), -118.5(m), -125.7

IR (ATR): 3344, 2972, 1749, 1560, 1383, 1358, 1265, 1189, 1145, 1110, 1006, 901, 690cm⁻¹

HRMS (ESI Orbitrap) m/z: [M-H]⁻ Calcd for C₁₃H₇F₁₈N₂O₄ 597.0118; Found 597.0126

### Example 2: Synthesis of bis(1,1,1,3,3,3-hexafluoropropane-2-yl) (2,2,3,3,4,4,5,5-octafluorohexane-1,6-diyl) dicarbamate [8FHBC]

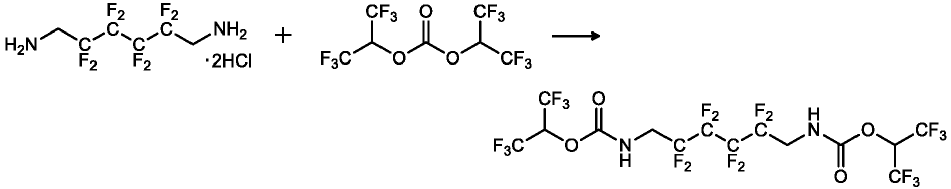

Bis(1,1,1,3,3,3-hexafluoropropane-2-yl)carbonate (BHFC) (10 mmol, 2.2 mL), 2,2,3,3,4,4,5,5-octafluorohexane-1,6-diamine hydrochloride (8FHDA·2HCl) (2.0 mmol, 0.67g), triethylamine (0.53 mmol, 3.8 mL) and a hydrofluoroether solvent ("Novec^{™} 7100" manufactured by 3M, 10 mL) as a solvent were added to a 50 mL round-bottom flask, and the mixture was stirred at room temperature for 2 hours. Then, 1 M hydrochloric acid, chloroform, the above hydrofluoroether solvent and water were added to the reaction mixture, and the organic phase and the aqueous phase were separated. The organic phase was dried over anhydrous sodium sulfate. The solvent was distilled away under reduced pressure, and the residue was dried in vacuo at 70°C for 2 hours to obtain a white solid as the target compound (0.92 g, 1.42 mmol, yield: 71%).

¹H NMR (400 MHz, CDCl₃, 293 K): δ/ppm: 5.68(sep, 2H, J=6.0Hz, CH), 5.40(t, J=6.6Hz, 2H, NH), 3.98(td, J=6.4, 15.2Hz, 4H, CH₂) ¹³C NMR (100 MHz, DMSO-d₆, 293 K) : δ/ppm: 153.3, 121.0 (q, ¹J_{C-F}=282.8Hz), 115.6(m), 111.0(m), 67.1(m), 41.1(m)

¹⁹F NMR (376 MHz, CDCl₃, 293 K) : δ/ppm: -73.6 (d, J=5.3Hz), - 118.8, -123.5(m)

IR (ATR) ν(cm⁻¹): 3342, 3094, 2981, 1736, 1560, 1438, 1382, 1360, 1257, 1201, 1155, 1106, 1081, 1014, 924, 906, 872, 751, 734, 691, 650

HRMS (ESI Orbitrap) m/z: [M-H]⁻ Calcd for C₁₄H₇F₂₀N₂O₄ 647.0092; Found 647.0083

### Example 3: Synthesis of polyurethane from 6FPBC and 1,6-hexanediol

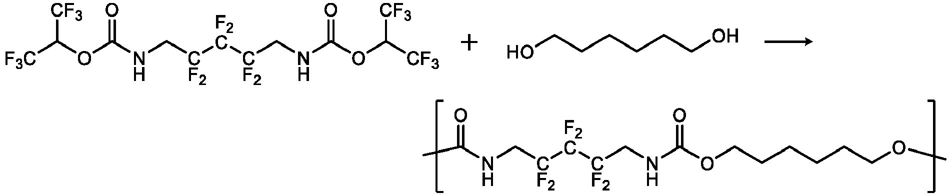

To a 10 mL round-bottom flask, 6FPBC (0.2 mmol, 0.12 g), 1,6-hexanediol (0.2 mmol, 0.024 g) and 1,4-diazabicyclo[2.2.2]octane (DABCO) (0.027 mmol, 0.003 g) were added. The mixture was stirred at 100°C for 67 hours.

The reaction mixture after the reaction was analyzed by infrared absorption spectroscopy. If there is an isocyanate in a reaction mixture, a strong signal must be observed in the range of 2200 to 2300 cm⁻¹. But a signal was not observed in the range as the following analytical result.

IR (ATR): 3321, 2931, 1702, 1550, 1262, 1143, 758 cm⁻¹

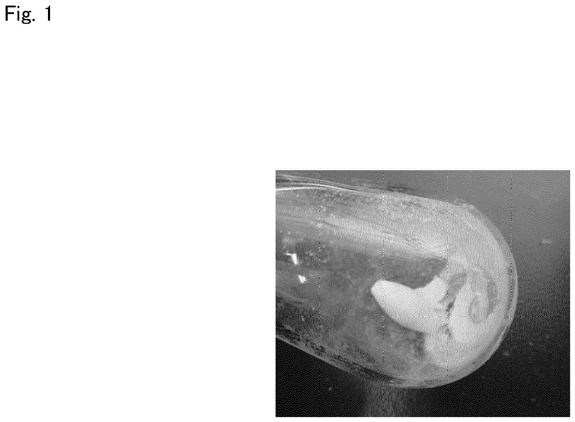

Then, the reaction mixture was dried in vacuo at 100°C for 2 hours to quantitatively obtain the target compound (0.08 g, 0.21 mmol [in monomer equivalent]). The analytical result of the product by infrared absorption spectroscopy was approximately the same as the analytical result of the reaction mixture after the reaction. In addition, a solvent that could dissolve the product could not be found, but at least the peak of the fluorine-containing alcohol was not observed by ¹H NMR analysis of the suspension and thus it was clear that the fluorine-containing alcohol could be removed from the target compound. Furthermore, the obtained target compound was a colorless white solid as Figure 1.

### Example 4: Synthesis of polyurethane from 6FPBC and PPG400

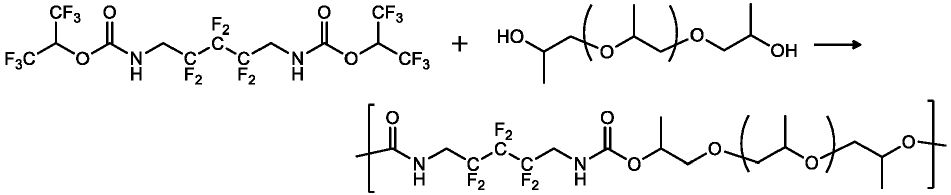

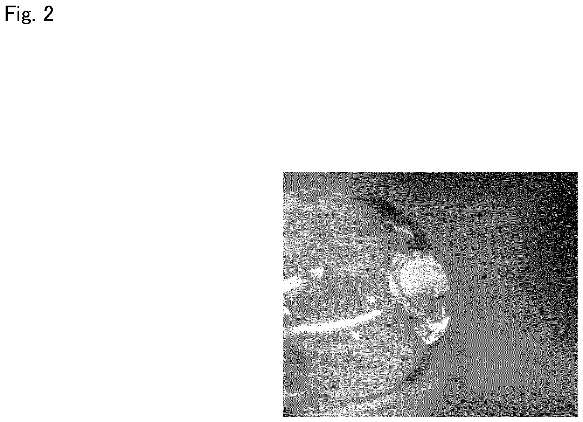

To a 10 mL round-bottom flask, 6FPBC (0.20 mmol, 0.12 g), polypropylene glycol (PPG, average molecular weight: 400) (0.2 mmol, 0.087 g) and 1,4-diazabicyclo[2.2.2]octane (DABCO) (0.027 mmol, 0.003 g) were added. The mixture was stirred at 100°C for 91 hours. Then, the mixture was dried in vacuo at 100°C for 2 hours to quantitatively obtain a pale yellow viscous liquid as the target compound (0.08 g, 0.26 mmol [in monomer equivalent]). The appearance photograph of the obtained target compound is shown as Figure 2. It was found that when PPG is used as the diol compound, the reaction temperature should be adjusted to be low or the reaction temperature should be adjusted to be short, since the reason why the target compound slightly became colored may be the decomposition of PPG.

¹H NMR (400 MHz, CDCl₃, 293 K): δ5.38(br., 2H, NH), 4.95(br., 2H, CH), 3.87(br., 4H, CH₂), 3.59-3.40(m, 22H, CH₂+CH), 1.25(d, 6H, J=6.4Hz, CH₃), 1.13(br., 18H, CH₃)

¹³C NMR (125 MHz, DMSO-d₆, 293 K): δ154.3, 75.5-75.0(m), 73.6-72.8(m), 72.4-71.9(m), 60.2-59.7(m), 47.9-47.6(m), 17.8, 17.6, 17.1

¹⁹F NMR (376 MHz, CDCl₃, 293 K, C₆F₆ as external standard) : δ-118.78(m, 4F, CF₂), -125.99(m, 2F, CF₂)

IR (ATR): 3328, 2974, 2935, 2873, 1726, 1541, 1455, 1376, 1352, 1278, 1254, 1139, 1100, 1070, 1014, 932, 776, 756 cm⁻¹ HPLC: M_{w}=21649, Mₙ=11195, M_{w}/Mₙ=1.93

### Example 5: Synthesis of polyurethane from 8FHBC and PPG400 using base

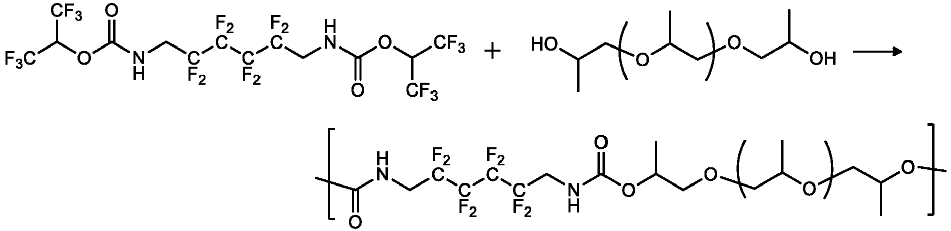

To a 7 mL test tube, 8FHBC (0.13 g, 0.20 mmol), PPG400 (80 mg, 0.20 mmol), DABCO (2.2 mg, 0.020 mmol) and 1,3-bis(trifluoromethyl)benzene (0.5 mL) were added. The mixture was stirred at 100°C for 35 hours. The reaction mixture after the reaction was analyzed by infrared absorption spectroscopy. A strong peak in the range of 2200 to 2300 cm⁻¹ characteristic of an isocyanate was not observed.

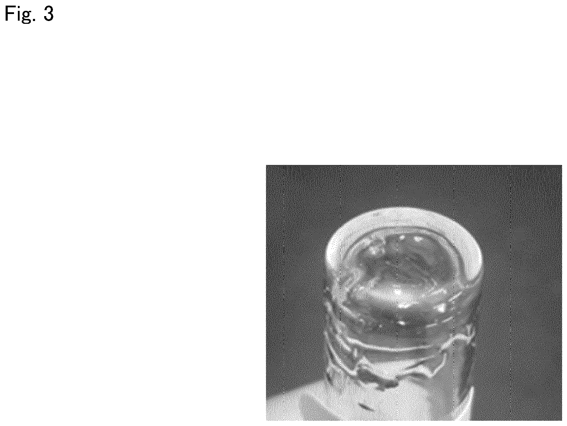

The solvent was distilled away from the reaction mixture under reduced pressure. Then, a colorless transparent viscous solid was obtained as the target compound (0.14 g, 0.19 mmol [in monomer equivalent], yield: 94%) by distillation under reduced pressure with using a glass tube oven to remove the base and drying at 50°C for 2 hours. The obtained polyurethane was analyzed by ¹H NMR and ¹⁹F NMR; as a result, the peak derived from hexafluoroisopropanol as the side product, specifically the peaks at 4.40 ppm and 3.06 ppm in ¹H NMR and the peak at -75.77 ppm in ¹⁹F NMR were not observed. It was therefore judged that the fluorine-containing alcohol as the side product did not remain in the produced polyurethane. The appearance photograph of the obtained target compound is shown as Figure 3.

¹H NMR (400 MHz, CDCl₃, 293 K): δ5.48(br., 2H, NH), 4.95(br., 2H, CH), 3.90(br., 4H, CH₂), 3.56-3.36(m, 22H, CH₂+CH), 1.25(d, 6H, J=6.4Hz, CH₃), 1.12 (br., 18H, CH₃)

¹⁹F NMR (376 MHz, CDCl₃, 293 K): δ-119.07 (m, 4F, CF₂), - 123.67(m, 4F, CF₂)

IR (ATR): 3309, 2974, 2937, 1713, 1542, 1379, 1258, 1163, 1106 cm⁻¹

HPLC: M_{w}=42,000, Mₙ=16,000, M_{w}/Mₙ=2.6

### Example 6: Synthesis of polyurethane from 8FHBC and PPG400 using base

The reaction was conducted in a similar condition to Example 5 except that the reaction time was extended from 35 hours to 47 hours; as a result, the molecular weight was further increased and thus an insoluble solid was obtained.

### Example 7: Synthesis of polyurethane from 8FHBC and PPG400 (1) Synthesis of bis(1,1,1,3,3,3-hexafluoropropane-2-yl) (2,2,3,3,4,4,5,5-octafluorohexane-1,6-dily)dicarbonate [8FHBC]

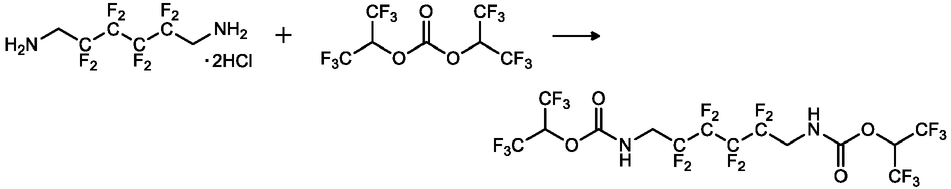

Bis(1,1,1,3,3,3-hexafluoropropane-2-yl)carbonate (BHFC) (30 mmol, 6.6 mL), 2,2,3,3,4,4,5,5-octafluorohexane-1,6-diamine hydrochloride (8FHDA·2HCl) (10 mmol, 3.33 g) and tetrahydrofuran (10 mL) as a solvent were added to a 100 mL round-bottom flask. Triethylamine (20 mmol, 2.8 mL) was added thereto, and the mixture was stirred at room temperature for 3 hours. Then, 1 M hydrochloric acid was added to the reaction mixture, and chloroform and water were added. The organic phase and the aqueous phase were separated. The organic phase was dried over anhydrous sodium sulfate. The solvent was distilled away under reduced pressure, and the residue was dried in vacuo at 70°C for 4 hour to obtain a white solid of 8FHBC (yield: 90%, amount: 5.83 g, 9 mmol). Thus, a general solvent can be used and an implementation scale can be increased in comparison with Example 2.

### (2) Synthesis of polyurethane

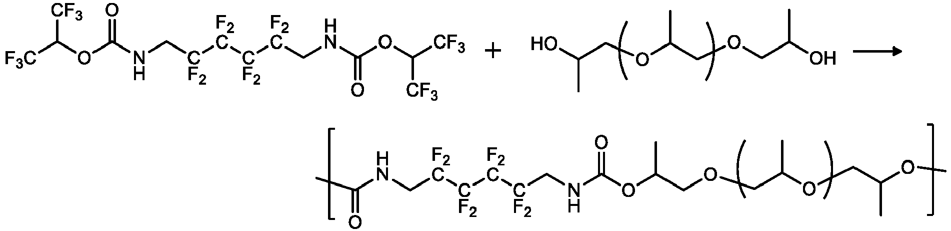

Polyurethane was synthesized similarly to Example 5 except that the reaction condition was changed to the reaction conditions shown in Table 1. The experimental example in the second column in Table 1 is Example 5. The yields are isolated yields. The reason why a molecular weight could not be determined in the experimental example in the third column is that polyurethane could not be dissolved in a solvent due to excessively large molecular weight.

**Table 1**

| Base (mmol) | Solvent (mL) | Reaction temp. | Reaction time | Yield | Mₙ | M_{w} | M_{w}/Mₙ |
|---|---|---|---|---|---|---|---|
| - | - | 190°C | 1h | quant. | 2,400 | 4,100 | 1.7 |
| DABCO (0.02) | 6F-Xylene (0.5) | 100°C | 35h | 94% | 16,000 | 42,000 | 2.6 |
| DABCO (0.02) | 6F-Xylene (0.5) | 100°C | 47h | 83% | unmeasurable | | |
| DABCO (0.02) | THF (0.5) | 60°C | 187h | quant. | 8,400 | 17,000 | 2.1 |
| K₂CO₃ (0.02) | THF (0.5) | 100°C | 43h | quant. | 4,700 | 9,200 | 2.0 |
| TEA (0.02) | THF (0.5) | 80°C | 186h | quant. | 1,600 | 3,100 | 2.0 |
| DABCO (0.02) | CH₃CN (0.5) | 80°C | 166h | quant. | 9,900 | 18,000 | 1.8 |
| TEA (0.02) | CH₃CN (0.5) | 100°C | 162h | quant. | 6,900 | 15,000 | 2.2 |
| DABCO (0.02) | Toluene (0.5) | 100°C | 195h | quant. | 19,000 | 44,000 | 2.3 |
| DABCO (0.02) | PhCN (0.5) | 100°C | 45h | quant. | 1,000 | 5,300 | 5.2 |

### Example 8: Synthesis of polyurethane from 8FHBC and ethylene glycol

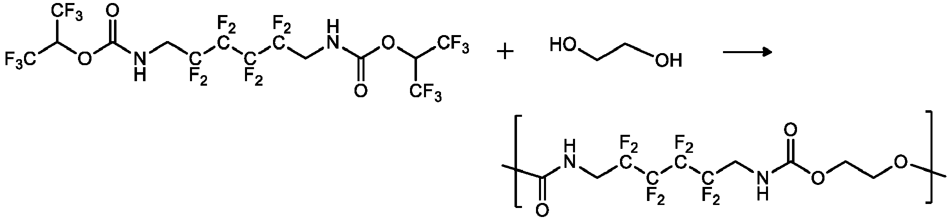

The solution prepared by dissolving 8FHBC (0.20 mmol, 0.13 g) and ethylene glycol (0.2 mmol, 11.2 µL) in tetrahydrofuran (0.5 mL) and 1,4-diazabicyclo[2.2.2]octane (DABCO) (0.02 mmol, 2.2 mg) were added into a 20 mL round-bottom flask, and the mixture was heated and stirred at 100°C for 43 hours. A precipitate that was hardly soluble in an organic solvent was produced and recovered by suction filtration to quantitatively obtain a white solid as the target compound

¹H NMR (400 MHz, DMSO-d₆, 293 K): δ/ppm=8.01(t, J=6.0Hz, 2H, NH), 4.19 (s, 4H, CH₂), 3.81 (td, J=16.4, 5.6Hz, 4H, CF₂CH₂)

¹³C NMR (100 MHz, DMSO-d₆, 293 K): δ/ppm=157.2, 156.8, 116.2(tt, ¹J_{C-F}=255.1Hz, ²J_{C-F}=29.2Hz), 111.5(tt, ¹J_{C-F}=2 61.7Hz, ²J_{C-F}=32.8Hz), 63.60, 59.3, 66.9, 40.91(t, ²J_{C-F}=21.9Hz)

¹⁹F NMR (376 MHz, DMSO-d₆, 293 K): δ/ppm=-118.12 (s, 4F, CF₂), - 123.47(s, 4F, CF₂)

IR (ATR): 3311, 3080, 2962, 1702, 1553, 1430, 1266, 1151, 1036, 737cm⁻¹

### Example 9: Synthesis of polyurethane from 8FHBC and diethylene glycol

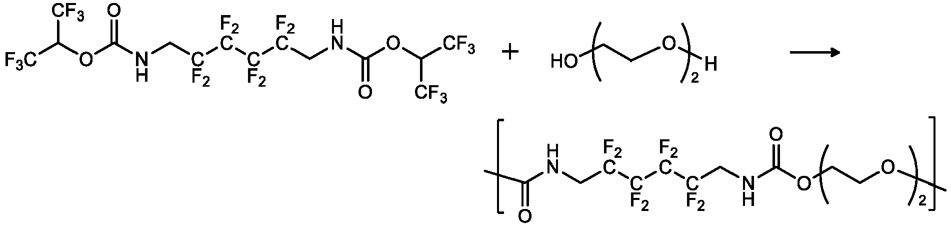

The solution prepared by dissolving 8FHBC (0.2 mmol, 0.13 g) and diethylene glycol (0.2 mmol, 21 mg) in toluene (0.5 mL) and 1,4-diazabicyclo[2.2.2]octane (DABCO) (0.02 mmol, 2.2 mg) were added into a 7 mL test tube having a screw mouth, and the mixture was heated and stirred at 100°C for 140 hours. Acetone was added to the reaction mixture to produce precipitate, and the precipitate was recovered by suction filtration and dried in vacuo at 100°C for 2 hours to quantitatively obtain a white solid as the target compound.

¹H NMR (400 MHz, DMSO-d₆, 293 K): δ/ppm=7.99(t, J=5.6Hz, 2H, NH), 4.12(t, J=4.0Hz, 4H, CH₂), 3.80(td, J=16.4, 6.0Hz, 4H, CF₂H₂), 3.60 (t, J=4. 4Hz, 4H, CH₂)

¹³C NMR (100 MHz, DMSO-d₆, 293 K): δ/ppm=157.01, 155.51, 116.2(tt, ¹J_{C-F}=2 54.4Hz, ²J_{C-F}=31.4Hz), 111.5(tt, ¹J_{C-F}=261.7Hz, ²J_{C-F}=32.8Hz), 69.05, 64.39, 40.90(t, J=22.6Hz)

¹⁹F NMR (376 MHz, DMSO-d₆, 293 K): δ/ppm=-118.02(s, 4F, CF₂), - 123.49(s, 4F, CF₂)

IR (ATR): 3322, 3088, 2951, 2886, 1701, 1550, 1428, 1262,

1164, 1149, 1118, 738 cm⁻¹

HPLC: M_{w}=22600, Mₙ=10300, M_{w}/Mₙ=2.2

### Example 10: Synthesis of polyurethane from 8FHBC and triethylene glycol

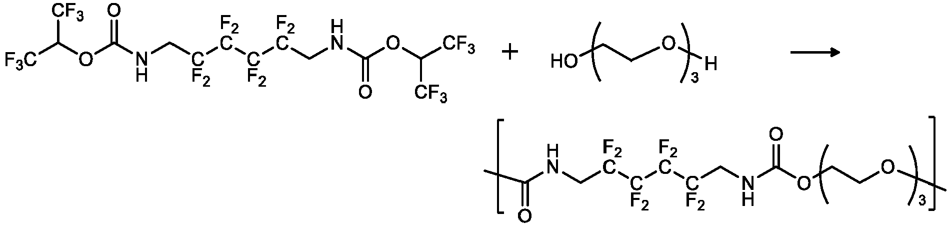

### (1) Use of THF as solvent

The solution prepared by dissolving 8FHBC (1.0 mmol, 0.65 g) and triethylene glycol (1.0 mmol, 150 mg) in tetrahydrofuran (1.0 mL) and 1,4-diazabicyclo[2.2.2]octane (DABCO) (0.1 mmol, 11 mg) were added into a 20 mL round-bottom flask, and the mixture was heated and stirred at 60°C for 331 hours. Acetone was added to the reaction mixture to produce precipitate, and the precipitate was recovered by suction filtration and dried in vacuo at 100°C for 2 hours to quantitatively obtain a white solid as the target compound (225 mg, 0.49 mmol [in monomer equivalent]).

HPLC: M_{w}=18000, Mₙ=10000, M_{w}/Mₙ=1.8

### (2) Use of toluene as solvent

The solution prepared by dissolving 8FHBC (0.2 mmol, 0.13 g) and triethylene glycol (0.2 mmol, 30 mg) in toluene (0.5 mL) and 1,4-diazabicyclo[2.2.2]octane (DABCO) (0.02 mmol, 2.2 mg) were added into a 7 mL test tube having a screw mouth, and the mixture was heated and stirred at 100°C for 140 hours. Acetone was added to the reaction mixture to produce precipitate, and the precipitate was recovered by suction filtration and dried in vacuo at 100°C for 2 hours to quantitatively obtain a white solid as the target compound (79 mg, 0.17 mmol [in monomer equivalent]).

¹H NMR (400 MHz, DMSO-d₆, 293 K): δ/ppm=7.98(t, J=6.0Hz, 2H,

NH), 4.11(t, J=4.4Hz, 4H, CH₂), 3.80(td, J=16.4, 6.4Hz, 4H, CF₂CH₂), 3.58(t, J=4.8Hz, 4H, CH₂) , 3.52(s, 4H, CH₂)

¹³C NMR (100 MHz, DMSO-d₆, 293 K): δ/ppm=157.03, 116.2(tt, ¹J_{C-F}=254.4Hz, ²J_{C-F}=33. 6Hz), 111.5 (tt, ¹J_{C-F}=264.7Hz, ²J_{C-F}=32.8Hz), 70.13, 69.11, 64.43, 40.89(t, J=22.6Hz)

¹⁹F NMR (376 MHz, DMSO-d₆, 293 K): δ/ppm=-118.16(s, 4F, CF₂), - 123.50(s, 4F, CF₂)

IR (ATR): 3323, 3080, 2955, 2902, 1704, 1546, 1428, 1266, 1153, 1122, 732 cm⁻¹

HPLC: M_{w}=8400, Mₙ=2900, M_{w}/Mₙ=2.9

### (3) Use of acetonitrile as solvent

The solution prepared by dissolving 8FHBC (0.2 mmol, 0.13 g) and triethylene glycol (0.2 mmol, 30 mg) in acetonitrile (0.5 mL) and 1,4-diazabicyclo[2.2.2]octane (DABCO) (0.02 mmol, 2.2 mg) were added into a 7 mL test tube having a screw mouth, and the mixture was heated and stirred at 80°C for 145 hours. Acetone was added to the reaction mixture to produce precipitate, and the precipitate was recovered by suction filtration and dried in vacuo at 100°C for 2 hours to quantitatively obtain a white solid as the target compound (yield: 74%, amount: 68 mg, 0.15 mmol [in monomer equivalent]) .

HPLC: M_{w}=5500, Mₙ=3200, M_{w}/Mₙ=1.7

### Example 11: Synthesis of polyurethane from 8FHBC and tetraethylene glycol

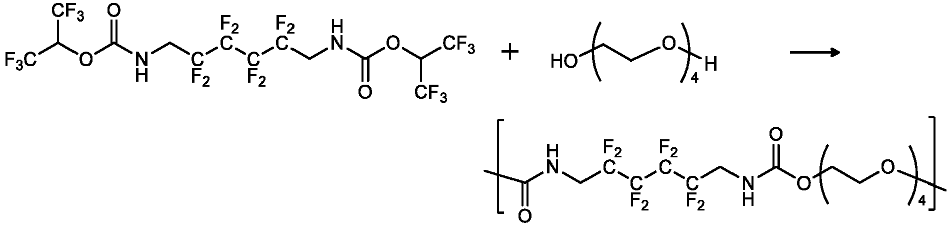

### (1) Use of THF as solvent

The solution prepared by dissolving 8FHBC (0.2 mmol, 0.13 g) and tetraethylene glycol (0.2 mmol, 34 µL) in tetrahydrofuran (0.5 mL) and 1,4-diazabicyclo[2.2.2]octane (DABCO) (0.02 mmol, 2.2 mg) were added into a 20 mL round-bottom flask, and the mixture was heated and stirred at 100°C for 43 hours. The reaction mixture was concentrated in vacuo at 100°C for 2 hours to obtain a colorless viscous liquid as the target compound (yield: 99%, amount: 100 mg, 0.199 mmol [in monomer equivalent]).

¹H NMR (400 MHz, CDCl₃, 293 K): δ/ppm=5.80(brs, 2H, NH), 4.27 (s, 4H, CH₂), 3.88 (t, J=12.0Hz, 4H, CF₂CH₂), 3.70-3.64(brs, 12H, CH₂)

¹³C NMR (100 MHz, CDCl₃, 293 K): δ/ppm=156.73, 155.86, 116.2(tt, ¹J_{C-F}=2 54.4Hz, ²J_{C-F}=33.6Hz), 111.5(tt, ¹J_{C-F}=264.7Hz, ²J_{C-F}=32.8Hz), 70.40, 70.10, 69.27, 64.57, 64.47, 41.08(t, J=24.1Hz)

¹⁹F NMR (376 MHz, CDCl₃, 293 K): δ/ppm=-119.12 (s, 4F, CF₂), - 123.74(s, 4F, CF₂)

IR(ATR): 3316, 3080, 2875, 1712, 1545, 1454, 1256, 1161, 1119, 937, 864 cm⁻¹

HPLC: M_{w}=5700, Mₙ=2600, M_{w}/Mₙ=2.2

### (2) Use of toluene as solvent

The solution prepared by dissolving 8FHBC (0.2 mmol, 0.13 g) and tetraethylene glycol (0.2 mmol, 39 mg) in toluene (0.5 mL) and 1,4-diazabicyclo[2.2.2]octane (DABCO) (0.02 mmol, 2.2 mg) were added into a 7 mL test tube having a screw mouth, and the mixture was heated and stirred at 100°C for 140 hours. The reaction mixture was concentrated in vacuo at 100°C for 2 hours to obtain a colorless viscous liquid as the target compound (yield: 89%, amount: 90 mg, 0.18 mmol [in monomer equivalent]).

HPLC: M_{w}=20000, Mₙ=6300, M_{w}/Mₙ=3.2

### Example 12: Synthesis of polyurethane from 8FHBC and 1,3-propanediol

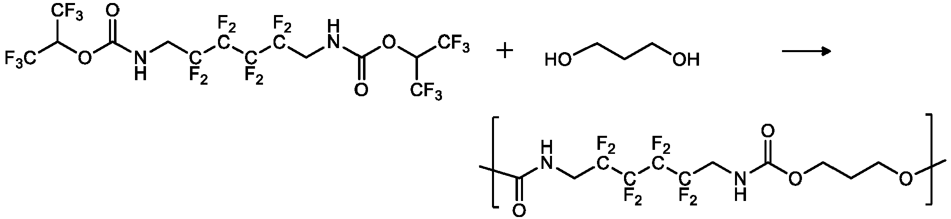

The solution prepared by dissolving 8FHBC (1.1 mmol, 0.74 g) and 1,3-propanediol (1.1 mmol, 86 mg) in toluene (1.0 mL) and 1,4-diazabicyclo[2.2.2]octane (DABCO) (0.11 mmol, 12.5 mg) were added into a 20 mL round-bottom flask, and the mixture was heated and stirred at 100°C for 209 hours. Acetone was added to the reaction mixture to produce precipitate, and the precipitate was recovered by suction filtration and dried in vacuo at 100°C for 2 hours to quantitatively obtain a white solid as the target compound (402 mg, 1.01 mmol [in monomer equivalent]) .

¹H NMR (400 MHz, DMSO-d₆, 293 K) : δ/ppm=7.92(t, J=6.0Hz, 2H, NH), 4.06(t, J=6.4Hz, 4H, CH₂), 3.80(td, J=16.0, 5.2Hz, 4H, CF₂CH₂), 1.87 (quin, J=6.4Hz, 2H, CH₂)

¹³C NMR (100 MHz, DMSO-d₆, 293 K): δ/ppm=156.99, 155.43, 116.2(tt, ¹J_{C-F}=2 54.4Hz, ²J_{C-F}=29.9Hz), 111.5(tt, ¹J_{C-F}=263.1Hz, ²J_{C-F}=33.5Hz), 61.84, 40.88(t, J=22.6Hz), 28.71

¹⁹F NMR (376 MHz, DMSO-d₆, 293 K): δ/ppm=-118.19(s, 4F, CF₂), - 123.51(s, 4F, CF₂)

IR (ATR): 3330, 3080, 2968, 2361, 1702, 1542, 1430, 1260, 1163, 1119, 1042, 742 cm⁻¹

HPLC: M_{w}=20600, Mₙ=6400, M_{w}/Mₙ=3.2

### Example 13: Synthesis of polyurethane from 8FHBC and polytetrahydrofuran

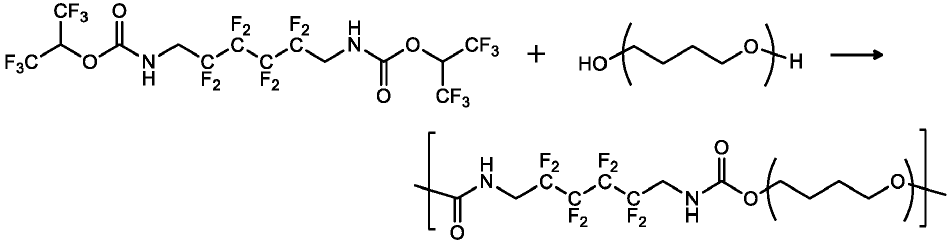

### (1) Use of toluene as solvent

The solution prepared by dissolving 8FHBC (5.0 mmol, 3.25 g) and polytetrahydrofuran (5.0 mmol, 1.25 g) in toluene (5.0 mL) and 1,4-diazabicyclo[2.2.2]octane (DABCO) (0.5 mmol, 56 mg) were added into a 20 mL round-bottom flask, and the mixture was heated and stirred at 100°C for 22 hours. Toluene (10 mL) was further added, and the mixture was further stirred for 22 hours. The reaction mixture was concentrated in vacuo at 100°C for 3 hours to quantitatively obtain a colorless transparent highly elastic solid as the target compound.

¹H NMR (400 MHz, CDCl₃, 293 K) : δ/ppm=5.36 (brs, 2H, NH), 4.12 (brs, 4H, CH₂) , 3.88 (t, J=14. 0Hz, 4H, CF₂CH₂), 3.42 (brs, 8.8H, CH₂) , 1.70-1.62 (brs, 12.8H, CH₂CH₂)

¹³C NMR (100 MHz, CDCl₃, 293 K) : δ/ppm=156.81, 155.87,

115.4(tt, ¹J_{C-F}=2 53.7Hz, ²J_{C-F}=30.6Hz), 111.3(tt, ¹J_{C-F}=263.9Hz, ²J_{C-F}=32.8Hz), 70.59, 70.08, 65.54, 41.10(t, J=22.6Hz), 26.38, 25.99, 25.73

¹⁹F NMR (376 MHz, CDCl₃, 293 K, C₆F₆ as external standard) : δ/ppm=-119.07 (s, 4F, CF₂), -123.68 (s, 4F, CF₂)

IR (ATR): 3323, 2943, 2859, 1700, 1541, 1374, 1256, 1153, 1120, 732 cm⁻¹

HPLC: M_{w}=191900, Mₙ=117500, M_{w}/Mₙ=1.63

### (2) Use of THF as solvent

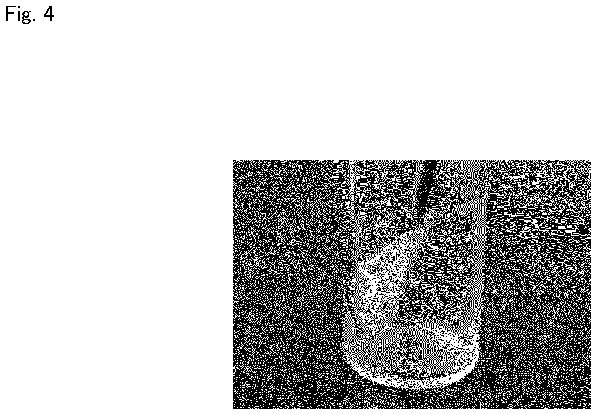

The solution prepared by dissolving 8FHBC (0.2 mmol, 0.13 g) and polytetrahydrofuran (0.2 mmol, 50 mg) in THF (0.25 mL) and 1,4-diazabicyclo[2.2.2]octane (DABCO) (0.02 mmol, 2.2 mg) were added into a 7 mL test tube having a screw mouth, and the mixture was heated and stirred at 60°C for 353 hours. The reaction mixture was concentrated in vacuo at room temperature for 2 hours to quantitatively obtain a colorless transparent solid as the target compound. The appearance photograph of the obtained target compound is shown as Figure 4.

HPLC: M_{w}=36400, Mₙ=18300, M_{w}/Mₙ=2.0

### Example 14: Synthesis of polyurethane from 8FHBC and hexafluoropentanediol

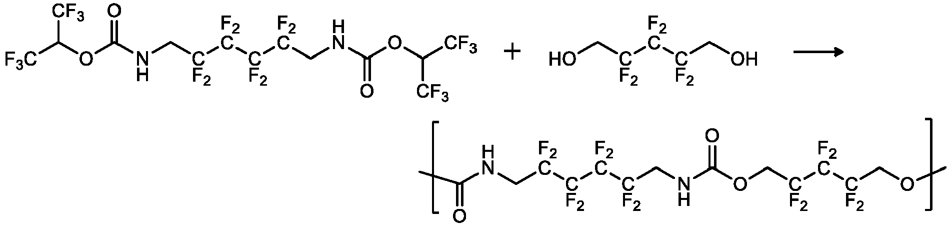

The solution prepared by dissolving 8FHBC (0.2 mmol, 0.13 g) and hexafluoropentanediol (0.2 mmol, 42 mg) in toluene (0.5 mL) and 1,4-diazabicyclo[2.2.2]octane (DABCO) (0.02 mmol, 2.2 mg) were added into a 7 mL test tube having a screw mouth, and the mixture was heated and stirred at 100°C for 21 hours. The reaction mixture was concentrated in vacuo at 100°C for 2 hours to quantitatively obtain a white solid as the target compound.

¹H NMR (400 MHz, DMSO-d₆, 293 K): δ/ppm=8.44(t, J=6.4Hz, 2H, NH), 4.75(t, J=14.0Hz, 4H, CH₂), 3.86(td, J=16.0, 6.0Hz, 4H, CF₂CH₂)

¹³C NMR (100 MHz, DMSO-d₆, 293 K): δ/ppm=155.62, 153.87, 118.99-108.31(m), 60.02(t, J=25.5Hz), 41.19(t, J=22.6Hz)

¹⁹F NMR (376 MHz, DMSO-d₆, 293 K, C₆F₆ as external standard) : δ/ppm=-118.05(s, 4F, CF₂), -119.46(s, 4F, CF₂), -123.43(s, 4F, CF₂), -125.55 (s, 2F, CF₂)

IR (ATR): 3343, 3092, 2980, 1724, 1552, 1433, 1262, 1149, 999, 738 cm⁻¹

HPLC: undetectable

### Example 15: Synthesis of polyurethane from 8FHBC and octafluorohexanediol

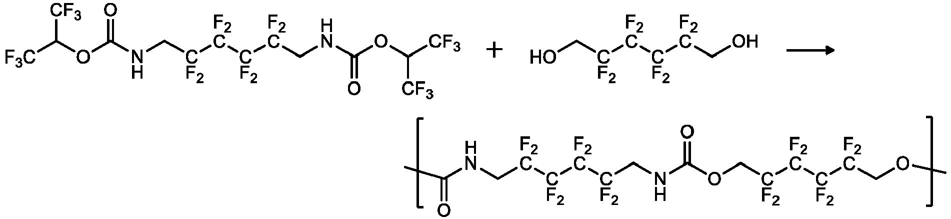

The solution prepared by dissolving 8FHBC (0.2 mmol, 0.13 g) and octafluorohexanediol (0.2 mmol, 52 mg) in toluene (0.5 mL) and 1,4-diazabicyclo[2.2.2]octane (DABCO) (0.02 mmol, 2.2 mg) were added into a 7 mL test tube having a screw mouth, and the mixture was heated and stirred at 100°C for 21 hours. The reaction mixture was concentrated in vacuo at 100°C for 2 hours to quantitatively obtain a white solid as the target compound.

¹H NMR (400 MHz, DMSO-d₆, 293 K): δ/ppm=8.45(t, J=6.0Hz, 2H, NH), 4.79(t, J=15.2Hz, 4H, CH₂), 3.86(td, J=16.4, 4.8Hz, 4H, CH₂)

¹³C NMR (100 MHz, DMSO-d₆, 293 K): δ/ppm=155.54, 153.79, 118.91-108.18(m), 59.96(t, J=24.8Hz), 41.20(t, J=22.6Hz)

¹⁹F NMR (376 MHz, DMSO-d₆, 293 K, C₆F₆ as external standard) : δ/ppm=-118.05(s, 4F, CF₂), -119.42(s, 4F, CF₂), -123.43(s, 8F, CF₂)

IR (ATR): 3346, 3084, 2976, 1725, 1540, 1434, 1263, 1118, 1004, 734 cm⁻¹

HPLC: M_{w}=25300, Mₙ=9400, M_{w}/Mₙ=2.68

### Example 16: Synthesis of polyurethane from 8FHBC and dodecafluorotetraethylene glycol

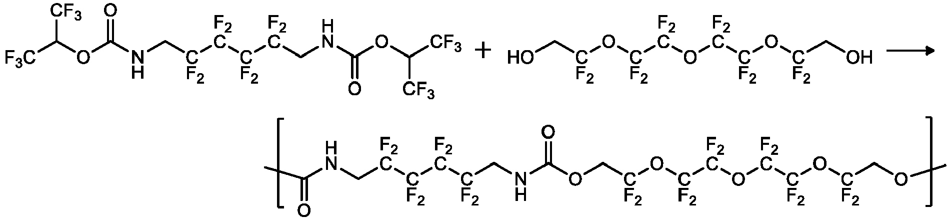

The solution prepared by dissolving 8FHBC (0.2 mmol, 0.13 g) and dodecafluorotetraethylene glycol (0.2 mmol, 82 mg) in toluene (0.5 mL) and 1,4-diazabicyclo[2.2.2]octane (DABCO) (0.02 mmol, 2.2 mg) were added into a 7 mL test tube having a screw mouth, and the mixture was heated and stirred at 100°C for 19 hours. The reaction mixture was concentrated in vacuo at room temperature for 2 hours to quantitatively obtain a white solid as the target compound.

¹H NMR (400 MHz, DMSO-d₆, 293 K): δ/ppm=8.42(t, J=4.8Hz, 2H,

NH), 4.71(t, J=8.0Hz, 4H, CH₂), 3.83(td, J=14.8, 5.2Hz, 4H, CH₂)

¹³C NMR (100 MHz, DMSO-d₆, 293 K): δ/ppm=155.32, 153.60, 122.29(t, J=277Hz), 118.88-108.42(m), 61.69(t, J=32.1Hz), 41.12(t, J=24.0Hz)

¹⁹F NMR (376 MHz, DMSO-d₆, 293 K, C₆F₆ as external standard) : δ/ppm=-76.96(s, 4F, CF₂), -88.30(s, 4F, CF₂), -88.68(s, 4F, CF₂), -118.31 (s, 4F, CF₂), -123.62 (s, 4F, CF₂)

IR (ATR): 3341, 3088, 2981, 2359, 1726, 1552, 1411, 1106, 1000, 737 cm⁻¹

HPLC: M_{w}=34600, Mₙ=20100, M_{w}/Mₙ=1.72

### Example 17: Synthesis of polyurethane from 8FHBC and Bisphenol A

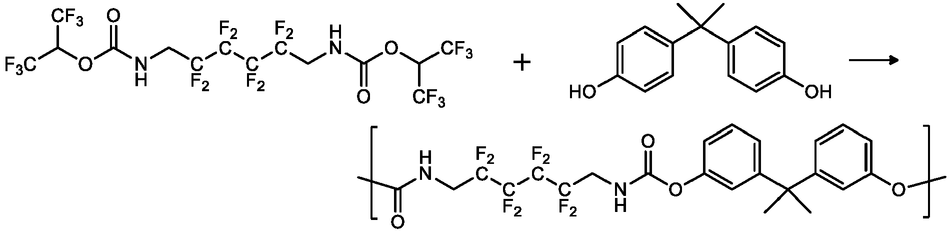

The solution prepared by dissolving 8FHBC (0.2 mmol, 130 mg) and Bisphenol A (0.2 mmol, 46 mg) in toluene (0.5 mL) and 1,4-diazabicyclo[2.2.2]octane (DABCO) (0.02 mmol, 2.2 mg) were added into a 7 mL test tube having a screw mouth, and the mixture was heated and stirred at 100°C for 19 hours. Acetone was added to the reaction mixture to produce precipitate, and the precipitate was recovered by suction filtration and dried in vacuo at 100°C for 2 hours to quantitatively obtain a white solid as the target compound (108 mg, 0.2 mmol [in monomer equivalent]).

¹H NMR (400 MHz, DMSO-d₆, 293 K): δ/ppm=8.51(t, J=6.0Hz, 2H,

NH), 7.21(dd, J=14.0, 8.8Hz, 4H, phenyl), 7.01(dd, J=14.0, 8.8Hz, 4H, phenyl), 3.91(td, J=16.8, 5.6Hz, 4H, CF₂CH₂),

1.64 (s, 6H, CH₃), 1.58 (s, 6H, CH₃)

¹³C NMR (100 MHz, DMSO-d₆, 293 K): δ/ppm=157.14, 155.06, 154.95, 140.19, 127.35, 127.29, 114.65, 118.89-107.46(m), 41.84, 40.65(t, J=22.6Hz), 30.60, 30.42

¹⁹F NMR (376 MHz, DMSO-d₆, 293 K, C₆F₆ as external standard) : δ/ppm=-117.83 (s, 4F, CF₂), -118.04 (s, 4F, CF₂), -123.34 (s, 4F, CF₂)

IR (ATR): 2970, 2358, 1732, 1614, 1542, 1499, 1212, 1169, 1120, 1017, 829 cm⁻¹

HPLC: M_{w}=8200, Mₙ=5100, M_{w}/Mₙ=1.6

## Claims

1. A method for producing a fluorine-containing polyurethane,
the method comprising the step of reacting a fluorine-containing biscarbamate compound represented by the following formula (I) and a diol compound represented by the following formula (II),
wherein the fluorine-containing polyurethane is represented by the following formula (III): 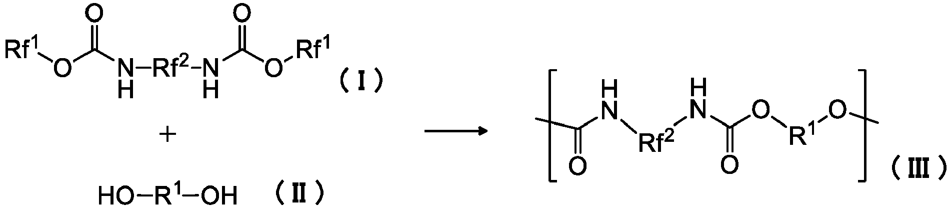 wherein
Rf¹ is a fluorine-containing C₁₋₆ alkyl group,
Rf² is a divalent fluorine-containing organic group,
R¹ is a divalent organic group.

2. The method according to claim 1, wherein the fluorine-containing biscarbamate compound represented by the formula (I) and the diol compound represented by the formula (II) are reacted under a temperature of 10°C or higher and 120°C or lower.

3. The method according to claim 1, wherein the Rf² is a group represented by the formula: CH₂-Rf³-CH₂ wherein Rf³ is a fluorine-containing C₁₋₈ alkane-diyl group.

4. The method according to claim 1, wherein the Rf² is a group represented by the formula: CH₂-(CF₂)ₙ-CH₂ wherein n is an integer of 1 or more and 8 or less.

5. The method according to claim 1, wherein the Rf¹ is CH(CF₃)₂.

6. The method according to any one of claims 1 to 5, wherein the fluorine-containing biscarbamate compound represented by the formula (I) and the diol compound represented by the formula (II) are reacted in the presence of a base.

7. A fluorine-containing biscarbamate compound represented by the following formula (I): 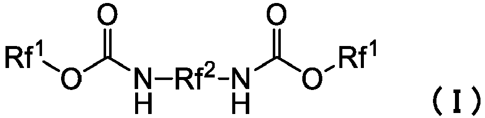 wherein
Rf¹ is a fluorine-containing C₁₋₆ alkyl group,
Rf² is a divalent fluorine-containing organic group.
